# EUROPEAN PATENT APPLICATION

(11) **EP 4 044 103 A1**
(43) Date of publication of application: **17.08.2022**
(21) Application number: 22156302.6
(22) Date of filing: 11.02.2022
(51) Int. Cl.: G06Q 50/22

(54) **SYSTEMS AND METHODS FOR GENERATING AND DELIVERING PSYCHOGRAPHICALLY SEGMENTED CONTENT TO TARGETED USER DEVICES**

(30) Priority: 11.02.2021 US 202163148528 P
(71) Applicant: PatientBond, Inc., Elmhurst, IL60126 (US)
(72) Inventor: FLOYD, II, David R., Elmhurst (US); WALKER, Brent Neil, Elmhurst (US); ALBERTSON, James Casey, Elmhurst (US); JUNEJA, Anurag, Elmhurst (US)
(74) Representative: Guardian IP Consulting I/S

(57) **Abstract**

A system for generating customizable messages is configured to (a) receive user seed information and a seed message from a client device; (b) determine a psychographic segment of a user associated with a user device based at least in part on the user seed information; (c) determine a delivery channel based on rules associated with the determined psychographic segment of the user; (d) generate a message to be communicated to the user device based on the psychographic segment and the delivery channel; and (e) send the message via the delivery channel to a user device. The message has a similar content to the seed message and having different wording from the seed message.

## Description

### TECHNICAL FIELD

The present disclosure relates generally to communicating messages to user devices, and more specifically, to adjusting messages based on psychographic segments for display or playback on the user devices.

### BACKGROUND

Engagement by patients is sometimes an important factor in the achieving positive health outcomes for the patients. Studies have shown that patients who are actively engaged in their health, wellness and healthcare experience better health outcomes. Patients who are active participants in their care and engage healthcare professionals, productively, are more likely to have care plan adherence to regular health screenings and checkups. They also typically exhibit healthy behaviors, such as exercise, proper nutrition and the avoidance of smoking or excessive drinking. Consequently, health issues are detected earlier or avoided altogether, leading to healthier people and lower costs to the healthcare system. There is a lack of systemic improved health outcomes in the U.S. compared to other countries. The U.S. is also associated with higher costs per person when compared to other countries. There is also limited success in effective patient engagement. Despite years of trying to improve patient engagement by employing dozens of technologies and tools and providers, systems, and payers spending millions of dollars, the reality is that robust patient engagement is still a challenge.

A challenge in engaging patients is that each individual processes information differently, and is receptive to differing types of information and modes of delivery. As such, the tailoring of messages to different patients can be beneficial in improving patient engagement. Whether written messages or messages provided over audio, standardizing message delivery in a networked environment can help improve patient engagement. The present disclosure is directed to solving problems related to patient engagement, displaying messages on electronic devices, and other technological issues associated therein.

### SUMMARY

According to some implementations of the present disclosure, a system for generating customizable messages is provided. The system includes a non-transitory computer-readable medium storing computer-executable instructions thereon such that when the instructions are executed, the system is configured to: (a) receive user seed information and a seed message (in electronic form) from a client device; (b) determine a psychographic segment of a user associated with a user device based at least in part on the user seed information; (c) determine an electronic delivery channel based on rules associated with the determined psychographic segment of the user; (d) generate a message to be communicated to the user device based on the psychographic segment and the delivery channel; and (e) send the message electronically or wirelessly via the delivery channel to a user device, the message having a similar content to the seed message and having different wording from the seed message.

In an implementation, the seed information includes demographics information such as age, race, income gender, or any combination thereof. In an implementation, the seed information includes behavioral information such as buying habits, browsing history, occupation, or any combination thereof. In an implementation, the psychographic segment is determined from a group including at least three psychographic segments. In an implementation, the group including at least three psychographic segments contains five psychographic segments. In an implementation, determining the psychographic segment of the user associated with the user device includes selecting a previously used psychographic segment for the user. In an implementation, the algorithmic rules associated with the determined psychographic segment of the user include a delivery channel rule indicating whether to communicate the message via a website, a mobile app, social media, SMS, email, phone call, or any combination thereof.

In an implementation, the rules associated with the determined psychographic segment of the user includes a psychographic segment rule indicating a number of messages to send to the user, a frequency of the messages to send to the user, a timeframe for sending the messages to the user, or any combination thereof. In an implementation, each message sent in the timeframe is sent through a different delivery channel. In an implementation, generating the message to be communicated to the user device includes swapping out an electronic image from the seed message with a first image that matches the determined psychographic segment. In an implementation, generating the message to be communicated to the user device includes automatically generating text using generative text models including Generative Pre-trained Transformer 3 (GPT-3) or a long short term memory (LSTM) neural network. In an implementation, the automatically generated text is edited to remove words based on an allowed list of words for the determined psychographic segment. In an implementation, the generated text is automatically edited to add words based on the allowed list of words. In an implementation, generating the message to be communicated to the user is further based on parameters associated with the user device. In an implementation, the user device is a smartphone and parameters associated with the user device include an operating system of the user device, a screen size of the user device, a pixel resolution of the user device, and/or a wake or sleep cycle of the user device. In an implementation, the user device is a desktop computer, a laptop computer, a tablet, a smartphone, a smart speaker, a telephone, or any combination thereof.

According to some implementations of the present disclosure, a method for generating customizable messages is provided. The method includes: (a) receiving, at an electronic server, user seed information and a seed message in electronic form from a client device; (b) determining, by the server, a psychographic segment of a user associated with a user's electronic device based at least in part on the user seed information; (c) determining, by the server, an electronic delivery channel based on rules associated with the determined psychographic segment of the user; (d) generating, by the server, an electronic message to be communicated over a computer network or wirelessly to the user device based on the psychographic segment and the delivery channel; and (e) sending, by the server, the message via the delivery channel to a user device, the message having a similar content to the seed message and having different wording from the seed message.

In an implementation, the seed information includes demographics information such as age, race, income gender, or any combination thereof. In an implementation, the seed information includes behavioral information such as buying habits, browsing history, occupation, or any combination thereof. In an implementation, the psychographic segment is determined from a group containing five psychographic segments.

### BRIEF DESCRIPTION OF THE DRAWINGS

The foregoing and other advantages of the present disclosure will become apparent upon reading the following detailed description and upon reference to the drawings.
FIG. 1 illustrates a block diagram of a system for communicating electronic messages to a user device for display on a graphical user interface thereon, according to some implementations of the present disclosure;
FIG. 2 illustrates information flow for generating the messages that will be communicated, according to some implementations of the present disclosure;
FIG. 3 is a flow diagram illustrating steps for communicating messages to the user device, according to some implementations of the present disclosure;
FIG. 4 illustrates a flow diagram for assigning images to different psychographic segments, according to some implementations of the present disclosure;
FIG. 5 illustrates an example psychographic segment distribution for a target population in a target geography, according to some implementations of the present disclosure;
FIG. 6 illustrates population density of an example psychographic segment in a geographic region, according to some implementations of the present disclosure; and
FIG. 7 illustrates an example message layout on the user device, according to some implementations of the present disclosure.

While the present disclosure is susceptible to various modifications and alternative forms, specific implementations have been shown by way of example in the drawings and will be described in detail herein. It should be understood, however, that the present disclosure is not intended to be limited to the particular forms disclosed. Rather, the present disclosure is to cover all modifications, equivalents, and alternatives falling within the spirit and scope of the present disclosure as defined by the appended claims.

### DETAILED DESCRIPTION

In the U.S., there is limited success in effective patient engagement when health services try to motivate patients to be more involved and proactive in their healthcare needs. A Harvard Family research project found that only 4.7% of people respond to short message service (SMS) messages sent by a health practice to encourage advocacy or market products. The research project also found that only 0.8% of people responded to the same message via email. Consequently, care plan adherence suffers. For example, 25% of new prescriptions are never filled. Even when filled, up to 50% of patients with chronic conditions fail to take their medications as prescribed.

Since individuals process information differently, one cannot assume everyone with the same health condition thinks and acts alike, or responds to traditional patient engagement systems sufficiently. Patients are individuals, with distinct personalities, values and priorities. Two people may share common characteristics, such as health condition, demographic or socioeconomic variables, and they may even behave the same way, but their motivations may be different. If patients' personalities and motivations are different, an engagement message - whether clinical, educational or marketing - needs to also be different to activate desired behaviors. Furthermore, the display and communication of the message through technological means specific to the different individuals can help promote engagement. For example, mobile phones and smartphones are ubiquitous in today's society. If a patient's electronic device is able to display or play a message that is tailored for the specific patient, then there is a higher probability of increasing patient engagement.

Thus, embodiments of the present disclosure provide systems and methods that automatically transform content intended for one or more users of communication devices from an initial text to a form more appropriate for the communication devices. The systems and methods utilize a psychographic segmentation model that includes at least three distinct psychographic segments (e.g., five psychographic segments, seven psychographic segments, ten psychographic segments, etc.). User seed information (e.g., any associated feature tied to a patient) can be used to determine the psychographic segment that the patient belongs. The psychographic segment, along with the communication device associated with the patient, can be used to generate a message to be communicated to the patient. The message can be voice message, an email, a message displayed on an app running on the user's communication device, etc. Although embodiments of the present disclosure will be described in the context of healthcare, the scope of the disclosure is not limited to the healthcare environment. Any type of content can be provided to communication devices. For example, financial industry may want to communicate news to customers in an efficient manner so that the news is displayed or narrated in an appropriate format on the communication devices associated with the customers. Lawyers may wish to communicate information to their clients, storeowners may wish to communicate with different segments of their customer base, advertisers may wish to market products to their customer base, etc.

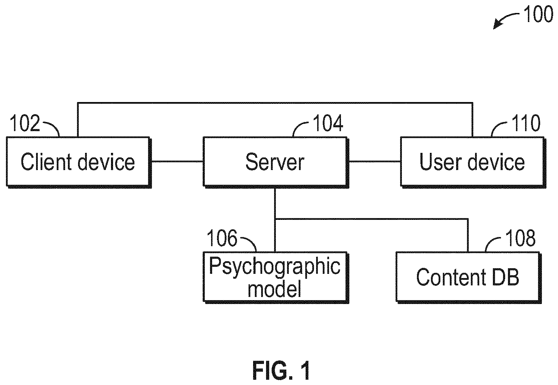

Referring to FIG. 1, a system 100 for communicating tailored messages to users is provided, according to some implementations of the present disclosure. The system 100 includes an electronic client device 102, an electronic server 104, an electronic user device 110, a psychographic computer model 106, and a content database (DB) 108. The client device 102 is a computing device for generating a seed message. In some examples, the client device 102 is or includes a desktop computer, a laptop computer, a tablet computer, a smartphone, etc. The client device 102 includes a processor and a memory that allows generating the seed message.

The server 104 is a computing device or computing system that houses an artificial intelligence model for generating messages to be communicated to the user device 110. The server 104 can be a remote server, a cloud server, a local server, and/or a networked server with physical infrastructure in multiple locations. The server 104 can include one or more computing devices networked together to perform functions ascribed to the server 104. The server 104 can include one or more processors, memory modules, hard drives, network cards, etc. In some implementations, the server 104 includes text to speech functionality and can thus communicate the message in an audio form.

The user device 110 is a computing device for receiving the electronic message generated by the server 104. The user device 110, in some implementations, is a telephone device. The user device 110, in some implementations, is similar to the client device 102 and includes one or more desktop computers, laptop computers, tablet computers, smartphones, etc. The user device 110 can further include wearable electronics, medical devices, or other health-related devices. For example, the user device 110 can be a glucose monitor, an activity tracker, a smartwatch, blood oxygen meter, or any other heal-related device with a display, speaker, and/or some other audio output. The user device 110, as identified in FIG. 1, corresponds to a communication device as described earlier (e.g., the communication device that a patient uses to receive information from health providers).

The psychographic model 106 and the content DB 108 are electronic databases or data stores, which are computing devices or computing systems with one or more processors and memory devices utilized to store and index information for fast retrieval. The psychographic model 106 stores a psychographic segmentation model for a population. Psychographics pertain to people's attitudes, personalities and lifestyles and are the core to their motivations and communication preferences. In some implementations, the psychographic model 106 stores a computer model pertaining to healthcare consumer attitudes and behaviors (i.e., a healthcare psychographic model). The healthcare psychographic model can be a stable and consistent psychographic model. For example, the healthcare psychographic model can be validated by surveying a large sample population (e.g., between 2000 and 5000 individuals). The healthcare psychographic model can be kept up-to-date by continually obtaining ongoing insights of consumer healthcare attitudes, sources of health information, the role of provider in managing their health, patients' desired forms of digital engagement, preferred communication channels and desired frequency of communication, etc.

The content DB 108 stores content utilized by the server 104 in generating the message to be communicated to the user device 110. The content can include images, sample text, etc. In some implementations, the psychographic model 106 and the content DB 108 are a single database.

Embodiments of the disclosure will be described in a healthcare context. The present disclosure is not limited to the healthcare context, and the focus on healthcare is merely used for illustrative purposes. In the healthcare context, the psychographic model 106 essentially stores a representation of an understanding of patient values, beliefs and motivations. The server 104 can use the information stored in the psychographic model 106 to create messages for encouraging better engagement from the patient. In order to create effective engagement with any patient population at scale, the server 104 uses artificial intelligence to automatically generate and deliver messages to any of the psychographic segments in the psychographic model 106. Thus, the server 104 leverages data from several consumer research studies conducted over time, as well as patient response data from past engagement using psychographic insights. The server 104 uses millions of data points/insights on patient attitudes, beliefs, behaviors, motivations and communication preferences to generate appropriate messages to target health care populations in order to improve patient engagement and care plan adherence.

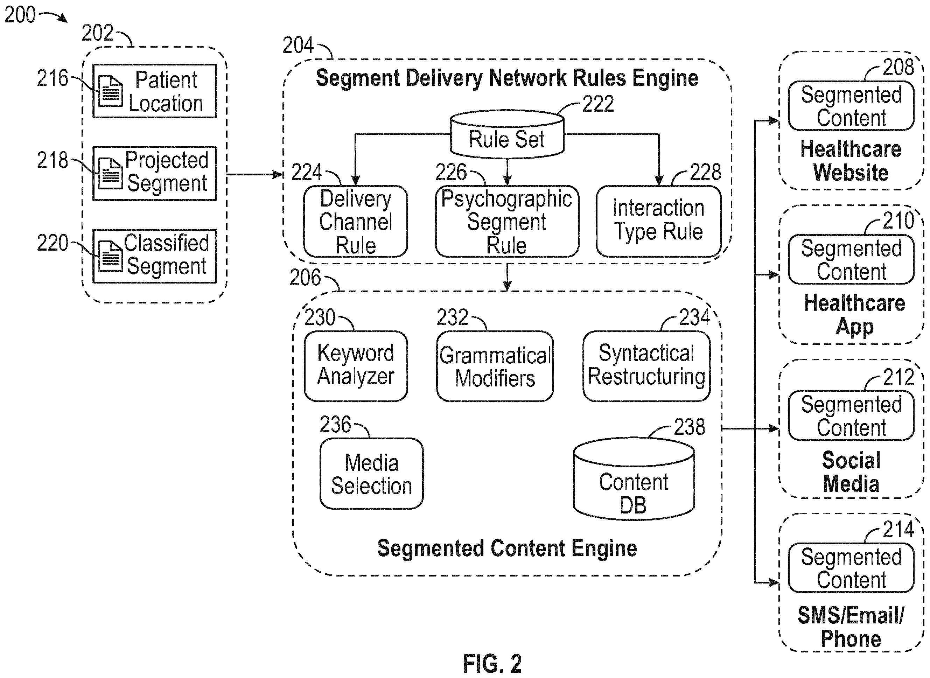

FIG. 2 illustrates information flow 200 for generating the electronic messages that will be automatically communicated to the user device 110, according to some implementations of the present disclosure. A segment classifier 202 can produce a psychographic segment so that a segment delivery network rules engine 204 can generate parameters for a segmented content engine 206. The segmented content engine 206 can then generated segmented content 208, 210, 212, and 214 based on the parameters. Each of the segment classifier 202, the segment delivery network rules engine 204, and the segmented content engine 206 can be implemented by the server 104 (FIG. 1). An engine is a combination of hardware and software configured to perform specific functionality.

The segment classifier 202 determines psychographic segment for a user using some user seed information. In some implementations, user seed information can include patient location 216. The user seed information can include demographics information (e.g., age, race, income, gender, etc.). The user seed information can include behavioral information (e.g., buying habits, browsing history, occupation, etc.). The user seed information can include information from web activity, interaction history (e.g., SMS replies, answered phone calls, etc.), health facility visit history, medication adherence, or other historical items.

The segment classifier 202 can determine the psychographic segment for the user as a projected segment 218 based on geofencing using the patient location 216, demographic information projections, or behavioral information projections. For example, the server 104 (FIG. 1) can determine the psychographic segment from seed information using information in the psychographic model 106. The segment classifier 202 can determine the psychographic segment for the user as a classified segment 220. For example, the client device 102 (FIG. 1) can choose a psychographic segment for the user, or a previously successfully projected psychographic segment for the user is reused based on the user's name.

In an implementation, neural networks are used to inspect datasets gathered from a healthcare organization. The healthcare organization may provide patients with a questionnaire, the answers to the questionnaire providing insight as to patients' psychographic segments. For example, the patients may be classified using a 12 question questionnaire upon admittance to the healthcare organization. Attributes from the healthcare system (e.g., electronic medical record (EMR), electronic health record (HER), or a claims database) can be used as user seed information. The user seed information can be used as inputs to the psychographic segmentation model running on the server 104, and the classified psychographic segment is the output of the psychographic segmentation model. Whenever a predictable pattern is established, and a prediction accuracy of 60% or greater is reached, the psychographic segmentation model can then be leveraged to determine the psychographic segment for any unseen records. The psychographic segmentation model can provide the predicted (or classified) psychographic segment. The psychographic segmentation model can provide an accuracy percentage based on the validation against unseen data in the seed model.

In an implementation, there are five distinct psychographic segments. Having five distinct psychographic segments is a good compromise because it provides more variation than three distinct psychographic segments but does not involve excessive computation or excessive segmentation of the population as a segmentation greater than five would involve. Having five distinct psychographic segments also helps with modeling the population in general without having too much overlap between preferences of the different segments. Five distinct psychographic segments were settled upon after experimentation with balancing computational resources with predictive capacity. A psychographic segment is a label or description of a particular psychographic dimension or set of dimensions or characteristics that a particular person can be categorized to fall within. For example, the five distinct psychographic segments can be labeled as: (1) self achievers, (2) balance seekers, (3) priority jugglers, (4) direction takers, and (5) willful endurers. Self achievers are proactive and driven by appearance. Self achievers are diligent with their treatment and screening. Self achievers are task oriented and challenge driven. Balance seekers are proactive and wellness-oriented. Balance seekers are open to ideas and options. Balance seekers have self-defined success. Balance seekers also look to health providers as resources. Priority jugglers are busy with things other than health. Priority jugglers are reactive to personal health. Priority jugglers are proactive with family health. Direction takers deeply trust healthcare providers. Direction takers visit doctors at first sign of an issue. Direction takers may not follow advice due to other responsibilities in their lives. Willful endurers live in the here and now. Willful endurers do what they like when they like. Willful endurers are self-reliant and resilient and only visit doctors when necessary. In some implementations, the five distinct psychographic segments when mapped to the U.S. population provides a distribution where about 19% of the population are self achievers, about 17% of the population are balance seekers, about 17% of the population are priority jugglers, about 15% of the population are direction takers, and about 31% of the population are willful endurers.

Psychographic segments can provide insights into a certain population's healthcare attitudes and the population's preferred sources for healthcare information. In some implementations, the psychographic segments are correlated with demographics. In some implementations, the psychographic segments provide insights into healthcare reform attitudes, health insurance provider satisfaction, health insurance desired benefits, etc. When looking at demographics, for example, age, psychographic segments highly correlated with age of individuals can provide insights into conditions experienced, medications currently being taken, and attitudes surrounding prescription or over-the-counter medication.

The five psychographic segments can exist on a continuum and provide different insights relating to an individual's attitude. For example, a direction taker may trust her doctor more than a balance seeker would. A direction taker may be on an extreme thinking that the doctor knows best while the balance seeker is on the other extreme thinking that she personally knows best more than the doctor would. Within that continuum, the self achiever would be closer to the direction taker's position, and the willful endurer would be closer to the balance seeker's position. The priority juggler would be in the middle of that continuum.

In another example, looking at the continuum between traditional medicine and alternative medicine, direction takers and priority jugglers would equally be on an extreme, having a highly favorable view of traditional medicine. Closer to the other extreme, balance seekers would be more favorable towards alternative medicine when compared to traditional medicine. The self achievers are closer to favoring traditional medicine but not to the extent of direction takers and priority jugglers, while the willful endurers are closer to the middle within the continuum of trusting traditional medicine vs. alternative medicine.

In another example, willful endurers can be very skeptical of healthcare and think of healthcare as "sick care" and can exist on one extreme of a healthcare attitude continuum. Self achievers on the other hand can think of healthcare as "wellness" at the other extreme of the continuum. The direction taker is closer in attitude to the willful endurer on this continuum, while the balance seeker is closer in attitude to the self achiever. The priority juggler's attitude toward healthcare is situated about in the middle of the continuum. As illustrated in these example, the psychographic segments can provide certain insights towards an individual's attitudes on various topics. These insights can help tailor messages to the specific individuals.

Once the segment classifier 202 determines a psychographic segment, the segment delivery network rules engine 204 uses a rule set 222 to determine a delivery channel rule 224, a psychographic segment rule 226, and an interaction type rule 228. As discussed above, different psychographic segments may prefer to receive information from different channels. For example, a self achiever is proactive with healthcare and so information provided on a healthcare website will likely be consumed by the individual. The delivery channel rule 224 can merely be that the information be provided on a healthcare website. There are different delivery channels considered, for example, a website, a mobile app, social media, SMS, email, phone call, etc.

The interaction type rule 228 includes a categorization for the message to be provided. For example, the interaction type rule 228 can include whether the message is a message reminding the patient of a health screening, a message reminding the patient of a regular health appointment, a message for chronic management of a condition (e.g., diabetes, high blood pressure, etc.), etc. The interaction type rule 228 can include specific types of exercises based on the categorization for the message. For example, some exercises may be more palatable based on psychographic segment, and the interaction type rule 228 can suggest appropriate exercises. For example, running two miles a day may be appropriate for a self achiever, but would be less palatable for a willful endurer.

The psychographic segment rule 226 can be chosen methods that have previously worked in the past for the specific psychographic segment determined by the segment classifier 202. For example, a willful endurer might need to have multiple reminders before taking healthcare actions. The psychographic segment rule 226 will include the number of reminders and how the reminders should be spaced out to avoid message fatigue. Communicating information to a user is not effective if the user does not read the message or merely ignores the message. In some implementations, a willful endurer may need to be provided with 4 total messages in the span of a week. The psychographic segment rule 226 for the willful endurer can provide that the first message be on the first day, and the delivery channel rule 224 can be that the first message be an email. The second message can be provided on the third day as a reminder on the healthcare app. The third message can be provided on the fourth day via social media. The fourth message can be provided on the sixth day via an SMS message. This messaging progression may be different for the self achiever where the psychographic segment rule 226 indicates two messages in the span of a week and the delivery channel rule 224 indicates that the first message be sent by email on the first day and the second message be sent by SMS on the fourth day.

The reminder messages in each of these examples are only sent if the individual has not completed the tasks indicated in the message. For example, if the messages are merely to inform the individual to go in for a screening, once the individual has set an appointment for a health screening then the reminders can be adjusted to remind of the time of the appointment. The reminders stop when the individual completes the health screening. In some cases, when the individual sets the appointment, then the reminders stop. The psychographic segment rule 226 can indicate a number of messages to send, a frequency of the messages, and/or a timeframe for sending the messages.

The message provided to the individual is tailored by the segmented content engine 206. The segmented content engine 206 includes a keyword analyzer 230, grammatical modifiers 232, syntactical restructuring 234, media selection 236, and a content DB 238. The content DB 238 is the same as or similar to the content DB 108 (FIG. 1). The message to be communicated to the individual is automatically restructured by the segment content engine 206 so that the message is easily digestible by the individual. Human behaviors are driven by values, feeling and beliefs so providing messaging that align with patient values and beliefs and/or messaging that resonate with patient priorities and preferences will be more convincing to the patient. Furthermore, if the message is viewed on a screen of the user device 110, then the visual arrangement of the message on screen can help with readability based on technical parameters associated with the user device 110.

The client device 102 can provide a seed message to be communicated to the user. The seed message can be adjusted by the segmented content engine 206 based on the channel delivery rule 224, psychographic segment rule 226, and interaction type rule 228 provided by the segment delivery network rules engine 204. The seed message can be in any unstructured format and can include pictures and/or words. The segmented content engine 206 analyzes keywords in the seed message using the keyword analyzer 230 to add, remove, and/or replace words.

For example, self achievers may be more receptive to words such as goals, measures, achiever, accomplish, win, beat, progress, overcome, expert, improve, best, etc. Self achievers may be less receptive to words such as relax, unmeasured, static, stasis, easy, lack of goals, lack of purpose, status quo, etc. A message tailored for a self achiever will avoid using certain words and proactively choose other words. In another example, balance seekers may be more receptive to words such as choices, options, knowledge, discover(y), explore, anticipate, interesting, proactive, enjoyable, entertaining, independent, invite questions, candid, etc. Balance seekers may be less receptive to words such as you must, required, feel (v. think), assume, strict, doctor says, rule (v. principle), no data, government program, etc. A message tailored for balance seekers will avoid using certain words and proactively choose other words. In another example, priority jugglers may be more receptive to words such as for the family, for work, dedication, devotion, duty, responsibilities, accountability, efficiency, be an example to others, etc. Priority jugglers may be less receptive to words such as alternative medicine, natural medicine, meditate, play, unpredictable, government program, taking a team approach to health, etc. A message tailored for priority jugglers will avoid using certain words and proactively choose other words. Direction takers may be more receptive to words such as credentials, professional, expertise, expert, easy, simple, institutional recommended, government recommended, fits your daily routine, etc. Direction takers may be less receptive to questions, unpredictable, unknown, risk, new routine, alternative medicine, natural medicine, change, etc. A message tailored for direction takers will avoid using certain words and proactively choose other words. Willful endurers may be more receptive to words such as convenient, cost-conscious, local, compassionate, simple, you deserve, take the first step, one step at a time, no hassle, fast service, etc. Willful endurers may be less receptive to words such as long term, many steps, future, challenge, involved process, multi-task, trust us (v. earning it), complex, etc. A message tailored for willful endurers will avoid using certain words and proactively choose other words.

The grammatical modifiers 232 can arrange or change text to ensure there are no grammatical errors. The grammatical modifiers 232 can use natural language processing and basic grammar rules to provide suggestions for subject-verb agreement, punctuation, etc. The segment content engine 206 can use an artificial intelligence model developed through supervised learning and stored in the content DB 238. The supervised learning models may be developed utilizing text categorization. These models combined with long short term memory (LSTM) text classification identifies syntax within the seed message that will not resonate well with a psychographic segment intended to receive the segmented content to be delivered (e.g., the segmented content 208). Keyword replacement by the keyword analyzer 230 followed by grammatical checks by the grammatical modifiers 232 are combined to provide replacement text and produce variant copies of the segmented content to be delivered.

The segment content engine 206 does not just swap words but can restructure sentences and the like. The syntactical restructuring 234 can rearrange sentences for better consumption by the psychographic segment determined by the segment classifier 202. The syntactical restructuring 234 performs sentence restructuring, leveraging keywords, synonyms, and antonyms to match sentences, paragraphs and phrases for replacement. The library containing the keywords, synonyms, and antonyms can be found in the content DB 238, a remote library, or an internet database or website. Alternate text generation is based intent of seed message, sentiment of seed message, and historical content proven effective with the psychographic segment that the message is being developed for. The historical content that was proven effective can be stored in the content DB 238. Syntactical restructuring 234 can use the interaction type rule 228 to add additional suggestions extrinsic to the seed message. For example, if the interaction type rule 228 includes a specific exercise recommendation for the determine psychographic segment the message is being generated for, then the exercise can be included by the syntactical restructuring 234.

The media selection 236 can swap images or other media for better conformity with a psychographic segment. The segmented content engine 206 provides as outputs the segmented content 208, 210,212, and/or 214. The segmented content 208, 210,212, and 214 are the messages to be communicated. In some implementations, the segmented content 208 is a longer message than the segmented content 214 or the segmented content 210. The user device 110 can enable scrolling functionality for the segmented content 208 but disable scrolling for the segmented content 210. Thus, parameters for the user device 110 can influence length and layout of messages. For example, a message delivered by text-to-speech can be shorter than a message delivered by email. In some cases, the user of the user device 110 can be impatient to robotic-sounding voice messages, so the duration of the segmented content 214 when delivered as audio is limited to about 10 seconds, 20 seconds, 30 seconds, etc. The content of the same message, when delivered by email, can be more detailed.

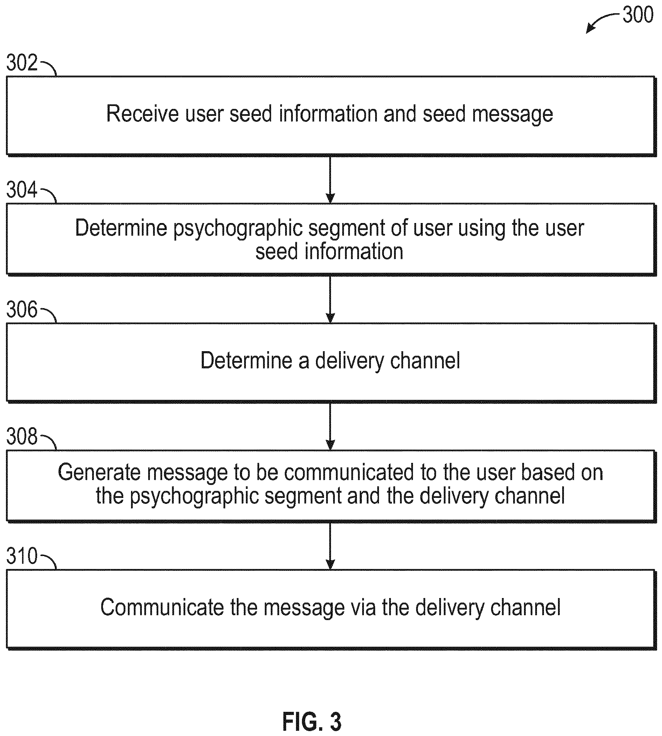

FIG. 3 is a flow diagram illustrating a process 300 for communicating messages to the user device 110 (FIG. 1), according to some implementations of the present disclosure. Steps in the process 300 can be performed by the server 104 (FIG. 1). At step 302, the server 104 receives user seed information and a seed message. In some implementations, the user seed information is received from the client device 102 as discussed above in connection with FIG. 2. The user seed information can include a name of the user, demographic information associated with the user, behavioral information associated with the user, location information associated with the user, a previous psychographic segment associated with the user, an income level associated with the user, a type of insurance carried by the user, etc. The seed message can be source content that is not segmented by psychographic segment. The seed message can be an email message that contains both text and images. The seed message can be a text file typed into a textbox on a graphical user interface of the client device 102. The seed message can be a message for encouraging the user to schedule a health screening. The seed message can be a single sentence or can be an excerpt spanning multiple paragraphs. The seed message can include a subject, a body, and a conclusion. The seed message can include a signature portion.

At step 304, the server 104 determines a psychographic segment for the user using the user seed information. The psychographic segment can be determined in a similar manner as discussed above in connection the segment classifier 202 of FIG. 2. The determined psychographic segment can be a projected segment for the user, a previous classification for the user, or a specific guess obtained by the client device 102. The psychographic model 106 can be leveraged in determining a projected segment for the user.

At step 306, the server 104 determines a delivery channel. The delivery channel can be determined in a similar manner as the segment delivery network rule engine 204, discussed above in connection with FIG. 2. For example, the psychographic segment determined at step 304 can have various rules association. One of these rules can be the delivery channel rule 224.

At step 308, the server 104 generates an electronic message to be communicated to the user device 110 based on the psychographic segment of step 304 and the delivery channel of step 306. The electronic message to be communicated is a modification of the seed message provided in step 302. The server 104 can generate the message using similar techniques as the segmented content engine 206, as discussed above in connection with FIG. 2. In some implementations, the server 104 can generate the message using OpenAI's Generative Pre-trained Transformer 3 (GPT-3) autoregressive language model using the seed message. In some implementations, the server 104 can use long short term memory (LSTM) neural networks to generate the message based on the seed message. In some implementations, text generated by a generative model (e.g., LSTM) is filtered and restructured as discussed above in connection with the keyword analyzer 230, the grammatical modifiers 232, and the syntactical restricting 234 of FIG. 2.

In some embodiments, given a single seed message, the generated message can look very different for users in different psychographic segments. For example, using the same seed message, a message generated for a self achiever may read as follows:
***Staying healthy is important to you,*** and we're ***committed to ensuring*** that you are seen for your scheduled appointment. Seeing your physician every 12 months gets you ***one step closer to your wellness goals,*** as it can not only keep you healthy, but could also possibly reduce your risk of heart disease and strokes.

A message generated for a balance seeker using the same seed message may read as follows:
***Living the life* you *want is important to you,*** and seeing your physician ***is a great choice*** for staying well and being healthy. ***Recent studies*** have linked heart attacks and strokes to diabetes, resulting from poor disease management. ***For more information,*** click (link).

A message generated for a priority juggler using the same seed message may read as follows:
We know ***you have many responsibilities,*** so we are sending you a ***courtesy reminder*** about your physician appointment - we will have everything set up and ready to go. ***With everyone that counts on you,*** you need to know that seeing your physician every 12 months can make a ***lifetime of difference*** in your health and wellness.

A message for a direction taker using the same seed message may read as follows: We ***would like to remind* you** of your upcoming appointment. Seeing your physician every 12 months ***is part of a good wellness routine.*** We wanted you to know that we have everything set up and ready to go, ***so please take a moment now*** to confirm.

A message for a willful endurer using the same seed message may read as follows: We are ***writing to remind you*** of your upcoming appointment. Seeing your physician every 12 months ***is a simple step*** that keeps you healthy and also possibly reduce your risk of heart disease and strokes, and ***making each day the best day it can be.***

In each of the above-generated messages, the highlighted portions indicate particular messaging that may have an emotional connection to the individual based on the psychographic segment that the individual resides.

In some implementations, the generated message can include an image in some implementations. An image included in the seed message can be analyzed in order to be swapped out for another image that might be more appropriate for the specific psychographic segment determined at step 304. For example, a self achiever is motivated by credentialed teams, so an image showing a doctor can be provided or can be more appropriate. A priority juggler is motivated by family and so an image showing multiple individuals at a dinner table or kids with adults, etc., can be provided or can be more appropriate. A direction taker is motivated by routine, so an image showing a single individual performing a task such as reading can be provided or can be more appropriate.

In some implementations, the generated message includes an "on-behalf-of' indication. For example, the server 104 can generate the message making it seem like the message was recommended by a credentialed individual (e.g., a specialist, a doctor, a nurse, etc.) or a family member or friend associated with the user. The "on-behalf-of' indication is based on a motivation quality associated with the psychographic segment that the user belongs. For example, a self achiever is motivated by credentialed individuals, so the generated message can include a name of a doctor known by the user, a name of a famous doctor, a name of the surgeon general, or a name of a healthcare facility. A priority juggler is motivated by family, so the generated message can include a name of a family member.

At step 310, the server 104 communicates the message to the user via the delivery channel determined at step 306. In some implementations, information associated with the user device 110 is available to the server 104. For example, information associated with the user device 110 can include a make and model of a smartphone, an operating system version of the smartphone, a screen size of the smartphone, a pixel resolution of the smartphone, wake or sleep cycles of the smartphone indicating times that the smartphone is in use, etc. The wake or sleep cycles can be used to tailor a schedule for when the message is communicated to the user device 110. For example, if the user seed information contains parameters of the user device 110 regarding when the user is most likely to use the user device 110, then the message can be delivered as an SMS at a moment indicated to be a wake cycle for the user device 110.

In some implementations, the user device 110 is a smart speaker or a personal assistance device with voice control. The server 104 can communicate the message as a notification to the user device 110 at night when the user may be asleep. That way, the smart speaker can light up a visual indicator of an unheard notification. This visual indicator will be a prompt for the user that an unheard message is queued up, and the user can listen to the message first thing in the morning.

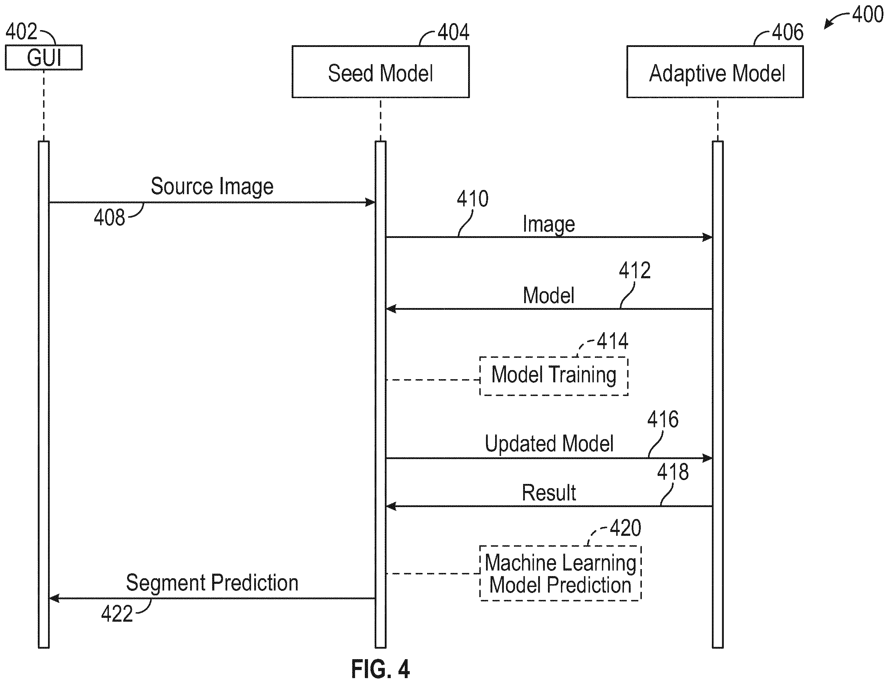

FIG. 4 illustrates a flow diagram 400 for assigning images to different psychographic segments, according to some implementations of the present disclosure. A graphical user interface (GUI) 402 of the client device 102 facilitates introducing a source image 408 to a seed model 404. The seed model 404 is an artificial intelligence model (e.g., a machine learning model) that resides on the server 104 and is used for predicting a psychographic segment that the source image 408 most resonates. The seed model 404 can reside in a storage (or memory) of the server 104 or can reside on the psychographic model 106 database. The server 104 formats the source image 408 into an image 410 for probing an adaptive model 406. The adaptive model 406 can also reside on the server 104 or any of the databases (e.g., the psychographic model 106 database and/or the content DB 106). The adaptive model 406 stores artificial intelligence models that can be updated over time based on source images introduced by the client device 102. In an example, the adaptive model 406 is initially trained using supervised learning so that the adaptive model 406 includes initial image resonation models for each psychographic segment (e.g., for each of the five psychographic segments identified above). The machine learning algorithm for training can be Keras as a service (AAS). Initially, the adaptive model 406 and the seed model 404 are identical. The seed model 404 and the adaptive model 406 are separated in FIG. 4 so that the adaptive model 406 can be retained as a persisted model or a backup in case. In some implementations, the seed model 404 and the adaptive model 406 are stored separately. The seed model 404 once trained, can be used to evaluate unseen images and determine classification of a presented source image (e.g., the source image 404). The adaptive model 406 becomes the new seed model, once verified through supervised learning.

The adaptive model 406 provides a model 412 to the seed model 404 in response to receiving the image 410 from the seed model 404. The model 412 is a prediction of the psychographic segment that corresponds to the source image 408. The source image 408 and the model 412 (i.e., prediction of the psychographic segment provided by the adaptive model 406) is stored for human review. So that during model training 414, the stored information is used for supervised learning where the prediction of the psychographic segment is corrected or approved. Any corrections to the prediction are persisted by updating the adaptive model 406 (i.e., updated model 416). A confirmation (i.e., result 418) is provided to the seed model 404. Ongoing improvement through supervised learning improves the accuracy of the prediction of images over time.

The seed model 404 then performs machine learning model prediction 420 on the image 410 to provide a segment prediction 422 to the GUI 402 of the client device 102. In some implementations, when the adaptive model 406 is updated with the updated model 416, the result 418 is the segment prediction 422 provided to the GUI 402. In some implementations, when the adaptive model 406 is not updated due to properly predicting the appropriate psychographic segment, the model 412 is provided as the segment prediction 422. FIG. 4 discloses two models, but it is envisioned that the seed model 404 and the adaptive model 406 can be implemented as one machine learning model, and the seed model 404 is merely the one machine learning model at a first timestamp, and the adaptive model 406 is the one machine learning model at a second timestamp.

In an example, the flow diagram 400 in FIG. 4 allows using supervised learning to build initial image resonation models for each of the five psychographic segments. The trained models are then used to evaluate unseen images and determine a correct classification for the unseen images. A new image is uploaded, and the saved models predict the classification of the uploaded image. Additionally, the new image along with its prediction is saved for human review and revision as needed. Any corrections to the prediction are persisted and the updated version of the model is used on subsequent images. Ongoing improvement through supervised learning improves the accuracy of the prediction of images over time.

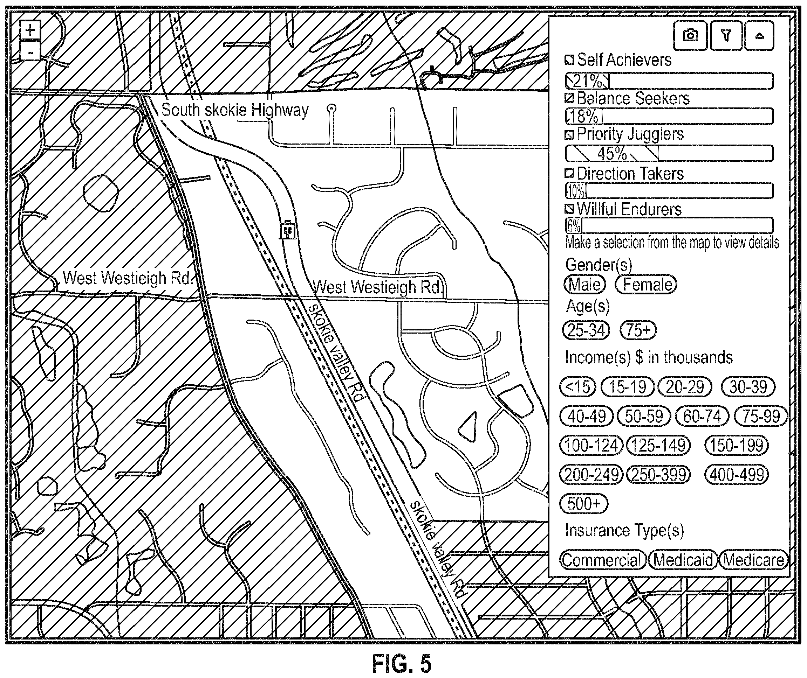

FIG. 5 illustrates segment distribution for a target population in a target geography filtered by age, gender, income and insurance. The target geography is shown as unshaded in FIG. 5. Within this target geography, there are estimated 21% self achievers, 18 balance seekers, 45% priority jugglers, 10% direction takers, and 6% willful endurers. The gender filter selection of male or female can change these percentages as the population is narrowed. Similarly, the filter selection of age range, income range, and/or insurance type can similarly change these percentages. FIG. 5 graphically shows that user seed information can be used to determine psychographic segment distribution in a geographical region. To determine the projected segment 218 (FIG. 2), several statistical tools may be employed. For example, the majority psychological segment can be chosen as the projected segment 218 for the user.

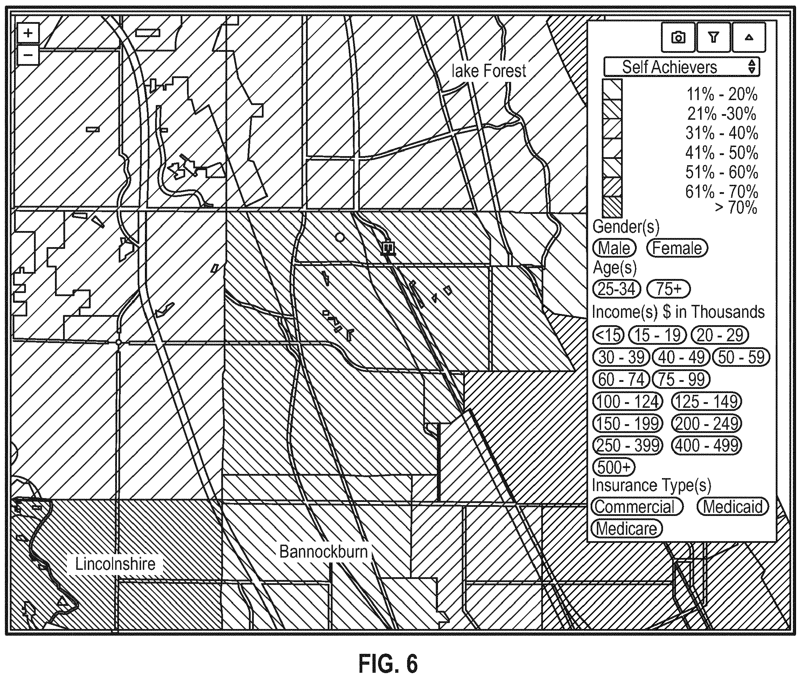

FIG. 6 illustrates population density of self achievers in a geographic region filtered by age, gender, income and type of insurance. Analogous to FIG. 5, geographic regions can be ranked based on psychographic segment. Self achievers with different distributions in different geographic regions are shown in FIG. 6. The filters for gender, age range, income range, and/or insurance types can be used to further narrow the population of interest to determine what percentage of that population are self achievers as a function of geographic region. The geographic regions can be shaded.

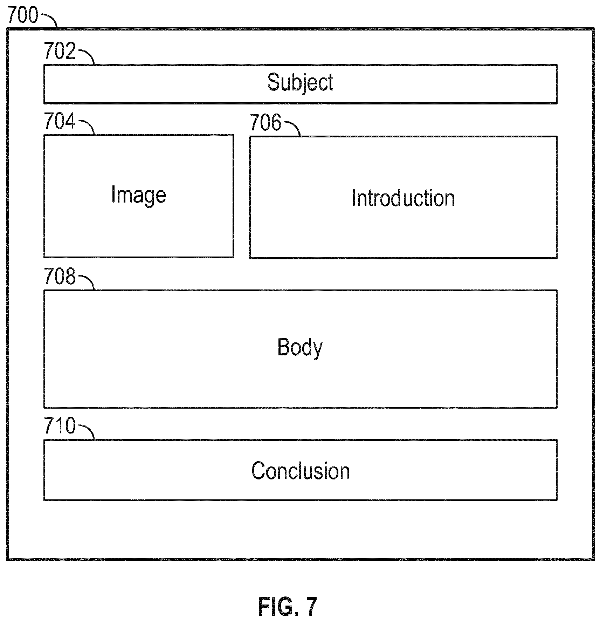

FIG. 7 illustrates an example message layout 700 on the user device 110, according to some implementations of the present disclosure. The message layout 700 can include a subject 702, an image 704, an introduction 706, a body 708, and a conclusion 710. In some implementations, the body 708 is kept the same for all psychographic segments while the subject 702, the image 704, the introduction 706, and the conclusion 710 are adjusted based on the psychographic segment.

In some implementations, the seed message of step 302 of FIG. 3 includes a seed message subject, a seed message image, a seed message introduction, a seed message body, and a seed message conclusion. After the server 104 performs the process 300 on the seed message, the seed message subject is transformed to the subject 702. The transformation of the seed message subject to the subject 702 can be based on an attention grabbing rule for the specific psychographic segment. The transformation of the seed message subject to the subject 702 can also be based on the pixel depth and pixel width of the user device 110. The subject 702 can include less words then the seed subject in order to fit larger text on the display of the user device 110. The seed image can be kept the same or swapped based on the psychographic segment so that the image 704 matches the psychographic segment of the user.

The seed introduction can be adjusted to the introduction 706 based on attention grabbing rule. Similarly, the seed conclusion 710 can be adjusted based on a goal to motivate. In regards to the parameters associated with the user device 110, the font and text can be chosen for display on the electronic video display of the user device 110. Parameters associated with the user device 110 can be used to limit length of text in the introduction 706 and the conclusion 710.

In some implementations, the seed message only contains the seed subject and the seed body, and the server 104 generates text for the introduction 706 and the conclusion 710, and modifies text for the subject 702 and the body 708. In some implementations, the user can have multiple smartphones or electronic devices with different screen sizes for displaying graphical user interfaces thereon. Embodiments of the present disclosure allow viewing a similar message on the multiple smartphones so that the similar message fits on an electronic screen or graphical user interface of each of the multiple smartphones without having to scroll. For example, the user can have a work smartphone with a 4.7-inch screen and a personal smartphone with a 6-inch screen. The user can install the same health app on both of these smartphones. The server 104 can generate a first message for work smartphone and a second message for the personal smartphone. The first message can be briefer can the second message since the 4.7-inch screen is smaller than the 6-inch screen. A user in a psychographic segment that does not like having too much information or that gets overwhelmed with too much information can be provided with information that is only limited to the screen size. As such, embodiments of the present disclosure can be used to automatically craft images for specific delivery channels so that no matter what form the seed message takes, the message layout 700 can automatically fit on the screen of the user device 110.

Five psychographic segments are provided as an example herein. In some implementations, the five psychographic segments are identified using an algorithm that assigns a series of coefficients to answers provided in a short survey completed by patients within a population. These psychographic segments differ according to their attitudes and behaviors with regard to health and wellness. By understanding these differences in health and wellness attitudes and behaviors, a healthcare provider (e.g., physician, nurse, pharmacist, hospital, health insurance plan, etc.) can better appeal to a patient's motivations through segment-specific messages and communication vehicles, to drive positive health behaviors, improve patient engagement and care plan adherence. The healthcare provider does not need to determine specific layout of messages for look and feel of how these messages will be presented on a patient's smartphone. Embodiments of the present disclosure can receive a seed message in any format and convert the message into a format that can be easily displayed on a screen of the patient's smartphone, taking into account readability parameters native to the smartphone of the patient.

Embodiments of the present disclosure provide a computer method that leverages years of psychographic consumer research data and insights, which can encompass gigabytes, terabytes, or even petabytes of electronically stored or archived data. The method includes using computerassisted artificial intelligence to process huge datasets beyond the scope of human capability and converting the analysis of those datasets to generate and deliver psychographically specific versions of patient engagement content. Embodiments of the present disclosure can significantly reduce the time and people resources required to design, produce and execute patient engagement communications. The method includes standardizing information display based on parameters and/or limitations associated with devices that the patients use in receiving the communication. The computer method can complement a healthcare provider's existing customer relationship management, electronic medical record or practice management systems.

Demographic data can be used in an artificial intelligence model to design tools/content. However, this model approach assumes that all members of a demographic subgroup (e.g., a specific gender, age group, ethnicity) think and act alike or are motivated by the same things. This is not true. Similarly, behavioral data (e.g., insurance claims data, hospital utilization data) can be used in an artificial intelligence model (using past behaviors to predict future behaviors) to design tools/content. However, two people can behave the same way (e.g., attended a wellness screening) but their motivations are different. Person A could have attended the wellness screening because she is highly proactive and wellness-oriented; Person B could be less involved in his health but attended the screening because his spouse encouraged him to. Both persons behaved the same way by getting the screening but their reasons were very different. Content to motivate them and influence their behavior would necessarily be different. As such, embodiments of the present disclosure provide a unique and effective computer method that uses psychographic data (attitudes, values, beliefs, priorities) in motivating patient behavior.

One or more elements or aspects or steps, or any portion(s) thereof, from one or more of any of claims 1-20 below can be combined with one or more elements or aspects or steps, or any portion(s) thereof, from one or more of any of the other claims 1-20 or combinations thereof, to form one or more additional implementations and/or claims of the present disclosure.

While the present disclosure has been described with reference to one or more particular embodiments or implementations, those skilled in the art will recognize that many changes may be made thereto without departing from the spirit and scope of the present disclosure. Each of these implementations and obvious variations thereof is contemplated as falling within the spirit and scope of the present disclosure. It is also contemplated that additional implementations according to aspects of the present disclosure may combine any number of features from any of the implementations described herein.

## Claims

1. A system for generating customizable messages, the system including a non-transitory computer-readable medium storing computer-executable instructions thereon such that when the instructions are executed, the system is configured to:
receive user seed information and a seed message from a client device;
determine a psychographic segment of a user associated with a user device based at least in part on the user seed information;
determine a delivery channel based on rules associated with the determined psychographic segment of the user;
generate a message to be communicated to the user device based on the psychographic segment and the delivery channel; and
send the message via the delivery channel to a user device, the message having a similar content to the seed message and having different wording from the seed message.

2. The system of claim 1, wherein the seed information includes demographics information such as age, race, income gender, or any combination thereof.

3. The system of claim 1 or claim 2, wherein the seed information includes behavioral information such as buying habits, browsing history, occupation, or any combination thereof.

4. The system of any one of claims 1 to 3, wherein the psychographic segment is determined from a group including at least three psychographic segments.

5. The system of claim 4, wherein the group including at least three psychographic segments contains five psychographic segments.

6. The system of any one of claims 1 to 5, wherein determining the psychographic segment of the user associated with the user device includes selecting a previously used psychographic segment for the user.

7. The system of any one of claims 1 to 6, wherein the rules associated with the determined psychographic segment of the user include a delivery channel rule indicating whether to communicate the message via a website, a mobile app, social media, SMS, email, phone call, or any combination thereof.

8. The system of any one of claims 1 to 7, wherein the rules associated with the determined psychographic segment of the user includes a psychographic segment rule indicating a number of messages to send to the user, a frequency of the messages to send to the user, a timeframe for sending the messages to the user, or any combination thereof.

9. The system of claim 8, wherein each message sent in the timeframe is sent through a different delivery channel.

10. The system of any one of claims 1 to 9, wherein generating the message to be communicated to the user device includes swapping out an image from the seed message with a first image that matches the determined psychographic segment.

11. The system of any one of claims 1 to 9, wherein generating the message to be communicated to the user device includes generating text using generative text models including Generative Pre-trained Transformer 3 (GPT-3) or a long short term memory (LSTM) neural network.

12. The system of claim 11, wherein the generated text is edited to remove words based on an allowed list of words for the determined psychographic segment.

13. The system of claim 12, wherein the generated text is edited to add words based on the allowed list of words.

14. The system of any one of claims 11 to 13, wherein generating the message to be communicated to the user is further based on parameters associated with the user device.

15. The system of claim 14, wherein the user device is a smartphone and parameters associated with the user device include an operating system of the user device, a screen size of the user device, a pixel resolution of the user device, and/or a wake or sleep cycle of the user device.
